# EUROPEAN PATENT SPECIFICATION

(11) **EP 2 231 061 B1**
(45) Date of publication and mention of the grant of the patent: **25.01.2017**
(21) Application number: 08859873.5
(22) Date of filing: 05.12.2008
(51) Int. Cl.: A61F 2/00

(54) **IMPLANTABLE PROSTHESIS**
IMPLANTIERBARE PROTHESE
PROTHÈSE IMPLANTABLE

(30) Priority: 07.12.2007 US 5738 P
(43) Date of publication of application: 29.09.2010
(73) Proprietor: C.R.Bard, Inc., Murray Hill, NJ 07974 (US)
(72) Inventor: KULLAS, Karen, E., Berkley MA 02779-1115 (US)
(74) Representative: Grey, Ian Michael
(86) International application number: PCT/US2008/013414
(87) International publication number: WO 2009/075786

(56) References cited:
- WO-A1-02/22047
- WO-A2-2006/020922
- WO-A2-2007/059199
- WO-A2-2007/109062
- US-A- 4 863 475
- US-A- 5 997 575
- US-A1- 2006 173 471
- US-A1- 2007 166 395
- US-B1- 6 319 264
- US-B1- 6 319 264
- US-B1- 6 610 006

## Description

### FIELD OF THE INVENTION

The present invention relates to prosthetic devices, and methods of using such devices for repairing, reconstructing, buttressing, or augmenting a defect or weakness in a wall of a tissue, muscle or organ.

### BACKGROUND OF THE INVENTION

Various prosthetic repair materials have been proposed to reinforce the abdominal wall and to close abdominal wall defects. One common approach to repair or reinforce a weakened abdominal wall employs a porous, knitted, synthetic mesh implant. Such a flexible and porous implant provides strength for reinforcing tissue and closing tissue defects, particularly in the abdominal cavity, while preserving sufficient openings in the material to support native tissue ingrowth. In certain procedures, however, the prosthetic mesh may come into direct contact with the sensitive abdominal viscera, potentially leading to postoperative adhesions between the mesh and, for example, abdominal contents, i.e., mesentery, bowel and intestines.

Various approaches to reducing the incidence of postoperative adhesions arising from the use of prosthetic mesh materials have been proposed by the prior art. One known approach employs a composite prosthesis, which combines a porous knitted mesh layer with a barrier material. Typically, the porous layer supports tissue ingrowth and provides mechanical strength as it bridges the defect while the barrier layer, which may principally be non-load bearing, reduces the potential for intestinal adhesions.

The earliest composite prostheses were entirely synthetic, resulting in a permanent implant. More recently, efforts to reduce the presence of foreign matter in the body have led to the development of at least partially biologic implants, in which biologic components are gradually resorbed by the body. One such biologic implant is the COLLAMEND [TM] implant available from C.R.Bard, Inc. of Murray Hill, New Jersey. The COLLAMEND [TM] material, formed of a single layer of acellular, lyophilized, crosslinked porcine dermis, is a fully resorbable implant exhibiting the required strength and durability for soft tissue reinforcement and tissue defect repair.

It is known from US2007/166395A1 to provide a prosthetic device for repairing or augmenting a tissue, muscle or organ defect, comprising a first portion of biological tissue that is remodelable, and configured for tissue ingrowth, said first portion constructed and arranged to cover or plug a defect; and a second portion of biological tissue that is more slowly enzymatically degradable after implantation than the first portion, and that is not configured for tissue ingrowth, said second portion having sufficient pullout strength so that said prosthetic device is fixatable through said second portion.

A prosthetic device according to the invention is characterised in that said first portion of biological tissue is acellular lyophilized porcine dermis that is not cross-linked or minimally cross-linked, and said second portion of biological tissue is acellular lyophilized porcine dermis that is cross-linked.

Preferred features of the present invention are defined in the dependent claims.

Other aspects, advantages and novel features will become more apparent from the following detailed description of embodiments of the invention when considered in conjunction with the accompanying drawings wherein:
FIG. 1 is an illustration of a composite two-layer resorbable prosthesis according to one embodiment of the present invention;
FIG 2. is an enlarged cross-sectional view taken along section line 2-2 of FIG. 1;
FIG. 3 is an illustration of a two-layer resorbable prosthesis according to another embodiment of the invention;
FIG. 4 is an enlarged cross-sectional view taken along section line 4-4 of FIG.3; and
FIG 5. is an illustration of the bottom side of the prosthesis shown in FIGS. 3 and 4.

### DETAILED DESCRIPTION

Aspects of the invention include a prosthetic device for repairing or augmenting a weakened or damaged tissue wall, muscle or organ (i.e., a defect), and a method of repairing or augmenting a weakened or damaged tissue wall, muscle or organ with a prosthetic device. Embodiments of the prosthesis may be particularly suited for procedures involving inguinal, femoral, hiatal, ventral, incisional and recurrent hernias, chest or abdominal wall reconstructions and augmentations, and the treatment of other large defects such as those that may occur in obese patients. The prosthetic device is not limited to a particular configuration and may, for example, be in the form of a patch, a plug, a patch and plug combination, or other arrangement. The prosthetic device may be employed in an open procedure, laparoscopic procedure, hybrid open or laparoscopic procedure, as well as other surgical methodologies and, in connection with abdominal wall procedures, may be performed preperitoneal or intraperitoneal.

The prosthetic device includes one or more portions or layers of acellular, lyophilized, porcine dermis Some aspects, or all, of a prosthetic device may include one or more of the following features: enzymatically degradable, remodelable, crosslinked, and/or configured for tissue ingrowth. Any of the foregoing properties, if provided, may be present uniformly throughout the prosthetic device or, instead, may vary at different locations of the implant. Further, the strength of the prosthetic device may substantially differ amongst disparate regions, with such strength being characterizable, for example, by a ball burst strength. The prosthetic device may be loaded, coated, impregnated or otherwise contain an anesthetic, antibiotic, drug or other medicament.

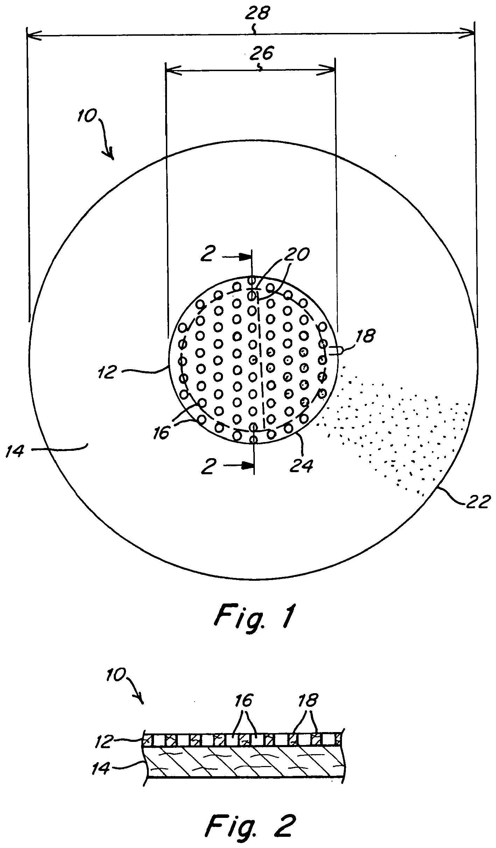

According to one embodiment of the invention, illustrated in FIGS. 1 and 2, a prosthesis 10 includes a first portion or layer 12 of biological tissue (e.g., autograft, allograft, xenograft, or combinations of any of the foregoing) that is configured to permit tissue infiltration and vascularization after implantation. The first portion or layer may be formed of acellular, lyophilized, porcine dermis having a thickness of between about 0.3 mm and 0.5 mm. The first layer or portion may be porous and/or may include a plurality of holes 16 that have been punched, die cut or otherwise formed therethrough to facilitate tissue ingrowth. The holes may have a diameter of about 0.060 to 0.094 inches and be spaced apart via a material web 18 between about 0.047 and 0.94 inches. It will be appreciated that the exact size, number, presence or absence, position and spacing of the holes may be varied to achieve the desired performance characteristics. Alternatively, or additionally, a surface of the ingrowth layer may be scraped, scored or otherwise treated to provide a surface texture that facilitates tissue ingrowth. Tissue infiltratable layer 12 is not crosslinked at all, or may be minimally crosslinked.

A second portion or layer 14 of the composite prosthesis also may be formed of a biological tissue. The second portion or layer may not be configured for tissue ingrowth; that is, the biological tissue may be substantially non-porous, not include any additionally formed openings therethrough, or otherwise not be receptive to tissue infiltration initially upon implantation (however, over time as the second portion is enzymatically attacked it may, ultimately, become susceptible to tissue ingrowth). The second portion or layer may be in the form of a barrier that does not stimulate adhesion formation when implanted. In applications where the second portion or layer includes, or is in the form of, a barrier, the barrier may be positioned to isolate the tissue infiltratable first portion or layer from sensitive tissue and organs that it may come into contact with, limiting the incidence of postoperative adhesions with the prosthetic device. Although not limited to a particular dimension, in certain embodiments the second portion or layer may have a thickness ranging from about 0.7 mm to 1.4 mm.

The second portion or layer may be sufficiently strong to prevent unwanted pullout of anchoring sutures, tacks, or other fixation constructs that may be used in a surgical procedure. The second portion or layer may be substantially stronger than the tissue infiltratable portion or layer, and may be characterized by a burst strength that is at least two times, three times, or four times greater than the burst strength of the first portion or layer. The second portion or layer is formed of acellular, lyophilized, cross-linked porcine dermis (such as COLLAMEND [TM]). This particular construction may serve as a barrier as it is not receptive to viscera adhesion. Also, the second portion or layer may include one or more through cuts or other openings to reduce the potential for fluid accumulation between the first and second layers. The second portion or layer is crosslinked.

The two portions or layers of the prosthesis 10 may be placed separately at the surgical site or may be in united form prior to implantation (for example, as supplied in sterile form by a medical device company). Connection of the two portions or layers preferably is by stitching 20 using resorbable suture material, for example, PDO or polydioxanone. Other mechanisms for connecting the two portions or layers, such as tacking, stapling, gluing and chemical bonding also are within the scope of the invention so long as the rendered prosthesis provides the desired performance characteristics. In certain embodiments, the rate of resorption may vary between the tissue infiltratable layer or portion and the second layer or portion, with the tissue infiltratable layer or portion resorbing more rapidly. For example, and without limitation, at least 50% of the tissue infiltratable portion or layer may be resorbable in less than twelve weeks after implantation, while at least 50% of the second portion or layer may be resorbable in no less than fifty-two weeks. In other embodiments, the second portion or layer may have a rate of resorption at least ten times longer, or twenty times longer, than the rate of resorption of the tissue infiltratable portion or layer. Resorbability, or rate or resorption, may be assessed in various way as should be apparent to one of skill in the art. Representative approaches include assessing either changes in weight or thickness, as well as by histological observation.

As shown in the figures, the two layers or portions may be joined face-to-face. In other embodiments, the two layers or portions may be joined edge-to-edge, where the adjoining edges are external edges, or may be an external edge adjacent an internal edge (for example, where a second portion or layer circumscribes a first portion or layer). In the edge-to-edge arrangements, the adjacent edges need not be contiguous; they may overlap or an intermediate structure may be provided therebetween. Similarly, in the face-to-face embodiment, an intermediate structure may separate the tissue infiltratable layer or portion and the second layer or portion. Other juxtapositions of the two layers or portions also are contemplated.

Although shown and described in the form of a patch, the prosthetic device is not limited to a particular configuration. Other contemplated arrangements, without limitation, include a plug and a patch/plug combination. In one embodiment, the tissue infiltratable layer or portion may be in the form of a plug and the second portion or layer in the form of an underlay, overlay or both an underlay and overlay. The plug may have a cylindrical, conical, or other three-dimensional shape, while the underlay and/or overlay may have, or may be arranged to have, a substantially planar, convex, concave, or other shape. The plug is minimally crosslinked or not crosslinked at all, while the underlay and/or overlay is crosslinked. Such a plug and patch combination may be surgically placed so that the plug resides in the defect, and the overlay (if provided) extends across an entrance to the defect and the underlay (if provided) extends across an exit to the defect. In any of the embodiments described herein, the prosthetic device may be imparted with flexibility as desired; for example, to facilitate handling, to conform to the defect, and/or to reduce detection of the device by the patient post implantation.

In certain embodiments, the tissue infiltratable portion or layer 12 is smaller in size than the second portion or layer 14, wherein the periphery of the second portion or layer 22 extends well beyond the outmost edge 24 of the tissue infiltratable portion or layer. For example, the smaller tissue infiltratable portion or layer may have a diameter 26 of about one-third to one-half to the diameter 28 of the second portion or layer. Configured this way, the centrally or otherwise located tissue infiltratable portion or layer may be positioned over a tissue gap or hernia defect, providing a matrix for accelerated ingrowth of bridging tissue. At the same time, the second portion or layer would extend sufficiently beyond the margins of the gap, and provide an accessible fixation location to permit secure attachment to surrounding tissue using adhesives or fixation constructs. Once secured at, or over, the repair site, the second portion or layer provides mechanical integrity and strength to the repair. In certain embodiments, a stronger second portion or layer compensates for the tendency of a flimsier tissue infiltratable portion or layer to bunch up, wrinkle or curl (as may occur where the tissue infiltratable portion or layer, alone, is attached directly to tissue surrounding the defect). Over time, the infiltratable portion or layer, the second portion or layer, and the resorbable sutures or other fixation mechanism will be resorbed by the body, with only native tissue remaining as a permanent repair of the defect.

In certain embodiments, the second portion or layer has a maximum width that is at least about twice the maximum width of the tissue infiltratable portion or layer. In other embodiments, the tissue infiltratable portion or layer and second portion or layer each have a substantially uniform thickness, and the second portion or layer is at least about twice the thickness of the tissue infiltratable portion or layer.

A representative procedure for forming a dual layer prosthesis, where the tissue infiltratable portion or layer is in the form of a sheet, and the second portion or layer also is in the form of a sheet, and the two sheets are joined face-to-face, according to the present invention will now be described. A piece of COLLAMEND [TM] porcine dermis, commercially available from C.R. Bard, Inc. of Murray Hill, New Jersey is die cut to form the tissue ingrowth sheet. A steel rule die is used to cut a 1.375 inch disk of material, and a plurality of 0.094 inch diameter, spaced, holes are formed, which are spaced apart with a web of 0.047 to 0.60 inches. A second piece of COLLAMEND [TM] material is then die cut to a diameter of 3.5 inches. No holes are placed in this layer. The smaller ingrowth layer may be placed centrally on the larger layer, or offset, and the layers are stitched together using a commercially available resorbable suture, such as PDO.

The representative embodiment of a composite implant includes a fenestrated, minimally or non-crosslinked, resorbable ingrowth layer attached to a microporous, resorbable, stronger and/or barrier layer. The fully crosslinked stronger and/or barrier layer has a diameter of 3.5 inches and a thickness of 0.039 inch. The ingrowth layer has a diameter of 1.375 inches and a thickness of 0.012 inch. It will be appreciated that the materials, degree of crosslinking, mesh diameters, thicknesses, hole size, position and spacing maybe varied in the foregoing example to suit particular needs.

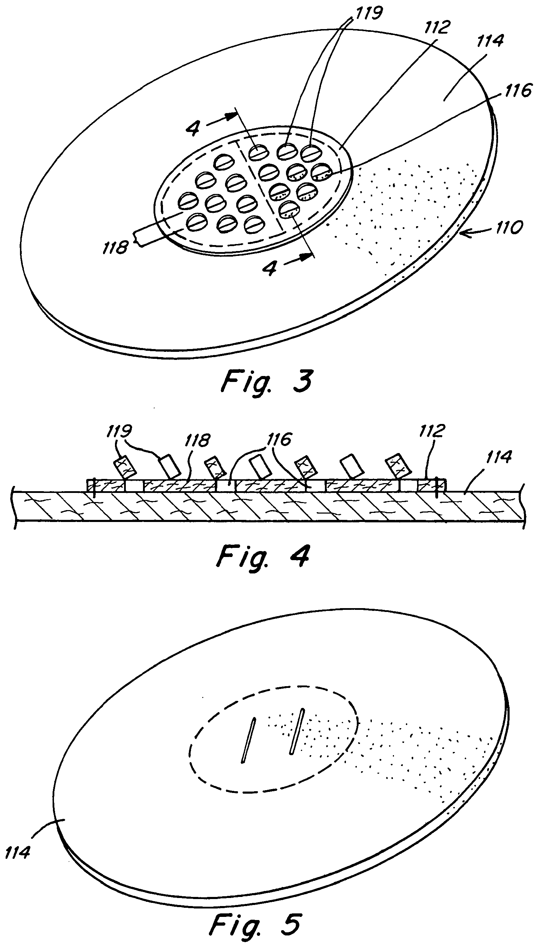

According to another embodiment of the invention, illustrated in FIGS. 3 through 5, a prosthetic implant 110 includes an ingrowth layer 112 formed of a porcine dermis material, which may be processed to be acellular and lyophilized or otherwise rendered suitable for implantation in a human or animal. Hinged tabs 119 are die cut or otherwise formed through the thickness of the ingrowth layer. The tabs 119 may then be folded or rotated out of the plane of the ingrowth layer forming openings 116 for tissue ingrowth. The tabs may be positioned to protrude into the plane of the tissue defect, providing an additional scaffold for ingrowth in the regions where the margins of the defect are not fully approximated. In the embodiment shown, tabs 119 have an arcuate or semi-circular profile. Advantageously, openings 116 provided in the ingrowth layer, encourage native tissue ingrowth and vascularization while reducing the potential for post-surgical seroma formation. It will be appreciated that the size, configuration, spacing and orientation of the tabs may be varied to meet particular needs. This prosthetic, and any of the other devices described herein, may additionally be impregnated, coated or otherwise configured with antibiotics, anesthetics, drugs, or other medicament; rendering, for example, a drug eluting prosthetic device.

### EXAMPLE

The following example is illustrative only and is not intended to limit the scope of the present invention. Burst strength characteristics were tested. Testing methodology and results appear below.

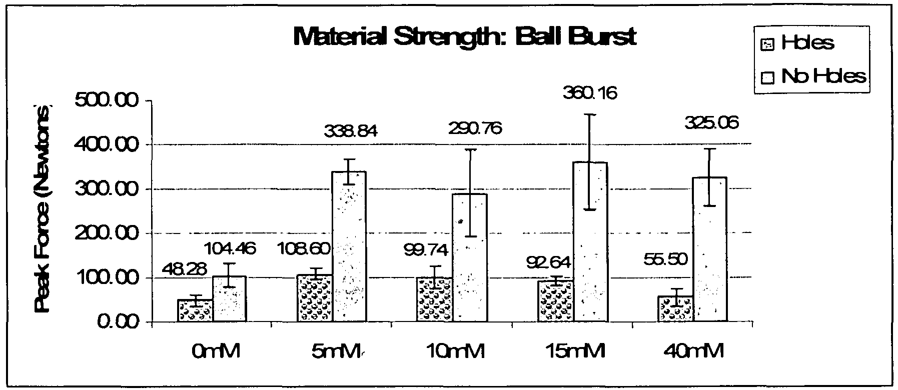

[0007] Ball burst strength was evaluated at five levels of crosslinking (0mM through 40 mM) of COLLAMEND [TM] material with and without holes. The results represent an average of at least 5 samples.

All samples were hydrated for 24 hours in 0.9% normal sterile saline prior to testing. All holes were 0.094-inches in diameter. A hole was formed in the center of the sample and five holes were located about the periphery at approximately 0.60 to 0.74-inches spacing. The samples were 1.5" circles. The ball burst testing followed, basically, the procedure recited in ASTM D3787-01, and utilized a 3/8-inch rod for bursting the samples in an Instron machine.

| Crosslink Concentration of EDAC | Peak Force (Newtons) Holes | Peak Force (Newtons) Holes st dev | Peak Force (Newtons) No Holes | Peak Force (Newtons) No Holes st dev |
|---|---|---|---|---|
| 0mM | 48.28 | 11.209 | 104.46 | 27.334 |
| 5mM | 108.60 | 13.185 | 338.84 | 29.852 |
| 10mM | 99.74 | 25.455 | 290.76 | 97.204 |
| 15mM | 92.64 | 11.095 | 360.16 | 107.930 |
| 40mM | 55.50 | 19.736 | 325.06 | 64.876 |

## Claims

1. A prosthetic device for repairing or augmenting a tissue, muscle or organ defect, comprising:
a first portion of biological tissue (12) that is constructed and arranged to cover or plug a defect; and
a second portion of biological tissue (14) having sufficient pullout strength so that said prosthetic device is fixatable through said second portion (14),
characterised in thatsaid first portion (12) of biological tissue is non-crosslinked or minimally crosslinked, and said second portion (14) of biological tissue is cross-linked, and in that said first and second portions of biological tissue are acelluar, lyophilized, porcine dermis.

2. The prosthetic device of claim 1, wherein said first portion (12) of biological tissue has a thickness of between about 0.3 mm and 0.5 mm.

3. The prosthetic device of claim 1, wherein said second portion (14) of biological tissue has a thickness of between about 0.7 mm and 1.4 mm.

4. The prosthetic device of claim 1, wherein said first and second portions (12,14) of biological tissue are in the form of a patch.

5. The prosthetic device of claim 4, wherein said patch includes a sheet of said second portion (14) of biological tissue that surrounds at least an aspect of a sheet of said first portion (12) of biological tissue.

6. The prosthetic device of claim 5, wherein said sheet of said first portion (12) of biological tissue is positioned against said sheet of said second portion (14) of biological tissue.

7. The prosthetic device of claim 6, wherein said sheet of said first portion (12) of biological tissue is positioned either facc-to-facc, or edge-to-edge, with said sheet of said second portion (14) of biological tissue.

8. The prosthetic device of claim 7, wherein said sheet of said first portion (12) of biological tissue is attached to said sheet of said second portion (14) of biological tissue by a resorbable material.

9. The prosthetic device of claim 1, wherein said second portion of biological tissue has a ball burst strength at least about twice that of said first portion (12) of biological tissue.

10. The prosthetic device of claim 9, wherein said second portion (14) of biological tissue has a ball burst strength of at least about three times that of said first portion (12) of biological tissue.

11. The prosthetic device of claim 9, wherein said second portion (14) of biological tissue has a ball burst strength of at least about four times that of said first portion (12) of biological tissue.

## Patentansprüche

1. Prothetische Vorrichtung für die Reparatur oder Augmentation eines Gewebe-, Muskel- oder Organdefekts, umfassend:
einen ersten Abschnitt aus biologischem Gewebe (12), der dazu aufgebaut und angeordnet ist, einen Defekt zu abzudecken oder zu stopfen; und
einen zweiten Abschnitt aus biologischem Gewebe (14), der ausreichende Ausziehfestigkeit aufweist, sodass die prothetische Vorrichtung durch den zweiten Abschnitt (14) hindurch fixierbar ist,
**dadurch gekennzeichnet, dass** der erste Abschnitt (12) aus biologischem Gewebe nicht vernetzt oder minimal vernetzt ist und der zweite Abschnitt (14) aus biologischem Gewebe vernetzt ist, und dadurch, dass es sich bei dem ersten und dem zweiten Abschnitt aus biologischem Gewebe um azelluläre, lyophiliserte Schweinedermis handelt.

2. Prothetische Vorrichtung nach Anspruch 1, wobei der erste Abschnitt (12) aus biologischem Gewebe eine Dicke von zwischen ungefähr 0,3 mm und 0,5 mm aufweist.

3. Prothetische Vorrichtung nach Anspruch 1, wobei der zweite Abschnitt (14) aus biologischem Gewebe eine Dicke von zwischen ungefähr 0,7 mm und 1,4 mm aufweist.

4. Prothetische Vorrichtung nach Anspruch 1, wobei der erste und der zweite Abschnitt (12, 14) aus biologischem Gewebe in Form eines Patchs sind.

5. Prothetische Vorrichtung nach Anspruch 4, wobei der Patch ein Flächengebilde aus dem zweiten Abschnitt (14) aus biologischem Gewebe umfasst, das mindestens eine Seite eines Flächengebildes aus dem ersten Abschnitt (12) aus biologischem Gewebe umgibt.

6. Prothetische Vorrichtung nach Anspruch 5, wobei das Flächengebilde aus dem ersten Abschnitt (12) aus biologischem Material an dem Flächengebilde aus dem zweiten Abschnitt (14) aus biologischem Gewebe anliegend positioniert ist.

7. Prothetische Vorrichtung nach Anspruch 6, wobei das Flächengebilde aus dem ersten Abschnitt (12) aus biologischem Material entweder Hauptfläche-an-Hauptfläche oder Rand-an-Rand an dem Flächengebilde aus dem zweiten Abschnitt (14) aus biologischem Gewebe anliegend positioniert ist.

8. Prothetische Vorrichtung nach Anspruch 7, wobei das Flächengebilde aus dem ersten Abschnitt (12) aus biologischem Material durch ein resorbierbares Material an dem Flächengebilde aus dem zweiten Abschnitt (14) aus biologischem Gewebe befestigt ist.

9. Prothetische Vorrichtung nach Anspruch 1, wobei der zweite Abschnitt aus biologischem Gewebe eine Kugelberstfestigkeit von mindestens ungefähr dem Zweifachen derjenigen des ersten Abschnitts (12) aus biologischem Gewebe aufweist.

10. Prothetische Vorrichtung nach Anspruch 9, wobei der zweite Abschnitt (14) aus biologischem Gewebe eine Kugelberstfestigkeit von mindestens ungefähr dem Dreifachen derjenigen des ersten Abschnitts (12) aus biologischem Gewebe aufweist.

11. Prothetische Vorrichtung nach Anspruch 9, wobei der zweite Abschnitt (14) aus biologischem Gewebe eine Kugelberstfestigkeit von mindestens ungefähr dem Vierfachen derjenigen des ersten Abschnitts (12) aus biologischem Gewebe aufweist.

## Revendications

1. Prothèse conçue pour réparer ou renforcer un défaut de tissu, de muscle ou d'organe, comprenant :
une première partie de tissu biologique (12) construite et agencée pour recouvrir ou boucher un défaut ; et
une seconde partie de tissu biologique (14) d'une résistance à l'arrachement suffisante pour que la prothèse puisse être fixée à travers ladite seconde partie (14),
**caractérisée en ce que** ladite première partie (12) de tissu biologique est non réticulée ou minimalement réticulée, et ladite seconde partie (14) de tissu biologique est réticulé, et **en ce que** lesdites première et seconde parties de tissu biologique sont un derme porcin acellulaire, lyophilisé.

2. Prothèse selon la revendication 1, dans laquelle ladite première partie (12) de tissu biologique a une épaisseur entre environ 0,3 mm et 0,5 mm.

3. Prothèse selon la revendication 1, dans laquelle ladite seconde partie (14) de tissu biologique a une épaisseur entre environ 0,7 mm et 1,4 mm.

4. Prothèse selon la revendication 1, dans laquelle lesdites première et seconde parties (12, 14) de tissu biologique ont la forme d'un timbre.

5. Prothèse selon la revendication 4, dans laquelle ledit timbre comporte une feuille de ladite seconde partie (14) de tissu biologique qui entoure au moins un aspect d'une feuille de ladite première partie (12) de tissu biologique.

6. Prothèse selon la revendication 5, dans laquelle ladite feuille de ladite première partie (12) de tissu biologique est positionnée contre ladite feuille de ladite seconde partie (14) de tissu biologique.

7. Prothèse selon la revendication 6, dans laquelle ladite feuille de ladite première partie (12) de tissu biologique est positionnée soit face-à-face, soit bord à bord, par rapport à ladite feuille de ladite seconde partie (14) de tissu biologique.

8. Prothèse selon la revendication 7, dans laquelle ladite feuille de ladite première partie (12) de tissu biologique est attachée à ladite feuille de ladite seconde partie (14) de tissu biologique par un matériau résorbable.

9. Prothèse selon la revendication 1, dans laquelle ladite seconde partie de tissu biologique a une résistance à l'éclatement d'au moins environ le double de celle de ladite première partie (12) de tissu biologique.

10. Prothèse selon la revendication 9, dans laquelle ladite seconde partie (14) de tissu biologique a une résistance à l'éclatement d'au moins environ trois fois celle de ladite première partie (12) de tissu biologique.

11. Prothèse selon la revendication 9, dans laquelle ladite seconde partie (14) de tissu biologique a une résistance à l'éclatement d'au moins environ quatre fois celle de ladite première partie (12) de tissu biologique.
